# FASCICULE DE BREVET EUROPEEN

(11) **EP 1 741 422 B1**
(45) Date de publication et mention de la délivrance du brevet: **01.04.2009**
(21) Numéro de dépôt: 06115250.0
(22) Date de dépôt: 09.06.2006
(51) Int. Cl.: A61K 8/89, A61K 8/06, A61Q 1/02, A61Q 19/00

(54) **Composition cosmétique comprenant un mélange d' élastomères**
Kosmetische Zusammensetzung enthaltend eine Mischung aus Elastomeren
Cosmetic composition comprising a mixture of elastomers

(30) Priorité: 04.07.2005 FR 0552022
(43) Date de publication de la demande: 10.01.2007
(73) Titulaire: L'Oréal, 75008 Paris (FR)
(72) Inventeur: Themens, Agnès, 92340, Bourg La Reine (FR)
(74) Mandataire: Boulard, Denis

(56) Documents cités:
- US-A- 6 103 250
- US-A1- 2002 022 040
- US-B1- 6 171 581

## Description

La présente invention a pour objet une composition cosmétique sous la forme d'une émulsion eau-dans-huile contenant des élastomères de silicone émulsionnants. La composition peut être une composition de maquillage ou de soin des matières kératiniques telles que la peau, les lèvres, des cheveux et notamment la peau. L'invention a également pour objet une procédé de maquillage ou de soin des matières kératiniques d'êtres humains

Les compositions de maquillage, en particulier les fonds de teint, peuvent se présenter sous forme de compositions anhydres ou d'émulsions, et de textures variées allant du fluide au solide.

Les émulsions à phase continue huileuse, ou émulsion eau-dans-huile, sont souvent préférées en raison de leur bonne adhérence à l'épiderme, leur capacité protectrice et leur capacité à former un film imperméable à l'eau. Le maquillage obtenu est confortable et ne dessèche pas la peau.
Cependant, ces émulsions procurent une sensation grasse au toucher, ce qui les rend peu attrayantes en particulier pour les utilisatrices ayant une peau à tendance grasse.

Pour obtenir une émulsion présentant une texture épaisse, il est connu d'utiliser des élastomères de silicone non émulsionnants comme décrit dans la demande EP 813403.

Toutefois, lorsque ces élastomères non émulsionnants, sont utilisés en quantité importante, notamment supérieure à 3 % en poids, la composition devient trop épaisse et présente même un aspect compact : la composition est difficile à étaler sur la peau et le maquillage déposé n'est pas homogène.

Or, les consommatrices sont à la recherche de nouveaux produits de maquillage ou de soin présentant des textures originales.

La présente invention a donc pour but de disposer de compositions de maquillage ou de soin s'étalant facilement sur la peau et ayant une texture épaisse qui reste crémeuse à l'application.

Une telle composition présente une texture crémeuse et une sensation de douceur lors de sa prise avec les doigts. Lors de son application sur la peau, la composition s'étire bien lors de son étalement et procure une sensation de douceur et de fondant.

Les inventeurs ont mis en évidence qu'en associant, dans une émulsion eau-dans-huile, au moins deux élastomères de silicone émulsionnants différents, il était possible d'obtenir une composition ayant une texture épaisse, crémeuse, s'étalant facilement sur la peau sans procurer de sensation grasse ou collante.

De façon plus précise, l'invention a pour objet une composition cosmétique sous forme d'émulsion eau-dans-huile comprenant au moins deux élastomères de silicone émulsionnants différents, la teneur totale en élastomères émulsionnants étant au moins supérieure ou égale à 3% en poids par rapport au poids total de la composition.

L'invention a également pour objet un procédé cosmétique (non thérapeutique) de maquillage ou de soin des matières kératiniques comprenant l'application sur lesdites matières kératiniques d'une composition telle que définie précédemment.

L'invention a encore pour objet l'utilisation d'une composition telle que définie précédemment pour obtenir un maquillage confortable, et/ou sans sensation de collant et/ou s'étalant facilement.

### Elastomères émulsionnants

La composition selon l'invention comprend au moins deux élastomères de silicone émulsionnants différents.

Par élastomère de silicone émulsionnant on entend un élastomère de silicone comprenant au moins une chaîne hydrophile.

L'élastomère de silicone émulsionnant est choisi d'une part parmi les élastomères de silicone polyoxyalkylénés.

L'élastomère de silicone polyoxyalkyléné est un organopolysiloxane réticulé pouvant être obtenu par réaction d'addition réticulation de diorganopolysiloxane contenant au moins un hydrogène lié au silicium et d'un polyoxyalkylène ayant au moins deux groupements à insaturation éthylénique.

De préférence, l'organopolysiloxane réticulé polyoxyalkyléné est obtenu par réaction d'addition réticulation (A1) de diorganopolysiloxane contenant au moins deux hydrogènes liés chacun à un silicium, et (B1) de polyoxyalkylène ayant au moins deux groupements à insaturation éthylénique, notamment en présence (C1) de catalyseur platine, comme par exemple décrit dans les brevets US5236986 et US5412004.

En particulier, l'organopolysiloxane peut être obtenu par réaction de polyoxyalkylène (notamment polyoxyéthyléne et/ou polyoxypropylène) à terminaisons diméthylvinylsiloxy et de méthylhydrogénopolysiloxane à terminaisons triméthylsiloxy, en présence de catalyseur platine.

Les groupes organiques liés aux atomes de silicium du composé (A1) peuvent être des groupes alkyles ayant de 1 à 18 atomes de carbone, tels que méthyl, éthyl, propyl, butyl, octyl, décyl, dodécyl (ou lauryl), myristyl, cétyl, stéaryl ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto.

Le composé (A1) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (C1) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Avantageusement, les élastomères de silicone polyoxyalkylénés peuvent être formés à partir de composés divinyliques, en particulier des polyoxyalkylènes ayant au moins deux groupes vinyliques, réagissant avec des liaisons Si-H d'un polysiloxane.

L'élastomère de silicone polyoxyalkyléné selon l'invention est de préférence mélangé avec au moins une huile hydrocarbonée et/ou une huile siliconée pour former un gel. Dans ces gels, l'élastomère polyoxyalkyléné est sous forme de particules non-sphériques.

Des élastomères polyoxyalkylénés sont notamment décrits dans les brevets US5236986, US5412004, US5837793, US5811487 dont le contenu est incorporé par référence.
Comme élastomère de silicone polyoxyalkyléné, on peut utiliser ceux commercialisés sous les dénominations "KSG-21", "KSG-20", "KSG-30", "KSG-31", KSG-32", "KSG-33", "KSG-210", "KSG-310", "KSG-320", "KSG-330", "KSG-340", "X-226146" par la société Shin Etsu, "DC9010", "DC9011" par la société Dow Corning.

L'élastomère de silicone émulsionnant est d'autre part choisi parmi les élastomères de silicone polyglycérolés.

L'élastomère de silicone polyglycérolé est un organopolysiloxane réticulé élastomère pouvant être obtenu par réaction d'addition réticulation de diorganopolysiloxane contenant au moins un hydrogène lié au silicium et de composés polyglycérolés ayant des groupements à insaturation éthylénique, notamment en présence de catalyseur platine.

De préférence, l'organopolysiloxane réticulé élastomère est obtenu par réaction d'addition réticulation (A) de diorganopolysiloxane contenant au moins deux hydrogènes liés chacun à un silicium, et (B) de composés glycérolés ayant au moins deux groupements à insaturation éthylénique, notamment en présence (C) de catalyseur platine.

En particulier, l'organopolysiloxane peut être obtenu par réaction de composé polyglycérolé à terminaisons diméthylvinylsiloxy et de méthylhydrogénopolysiloxane à terminaisons triméthylsiloxy, en présence de catalyseur platine.

Le composé (A) est le réactif de base pour la formation d'organopolysiloxane élastomère et la réticulation s'effectue par réaction d'addition du composé (A) avec le composé (B) en présence du catalyseur (C).

Le composé (A) est en particulier un organopolysiloxane ayant au moins 2 atomes d'hydrogène liés à des atomes de silicium distincts dans chaque molécule.

Le composé (A) peut présenter toute structure moléculaire, notamment une structure chaîne linéaire ou chaîne ramifiée ou une structure cyclique.

Le composé (A) peut avoir une viscosité à 25 °C allant de 1 à 50 000 centistokes, notamment pour être bien miscible avec le composé (B).

Les groupes organiques liés aux atomes de silicium du composé (A) peuvent être des groupes alkyles ayant de 1 à 18 atomes de carbone, tels que méthyl, éthyl, propyl, butyl, octyl, décyl, dodécyl (ou lauryl), myristyl, cétyl, stéaryl, ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto. De préférence, ledit groupe organique est choisi parmi les groupes méthyl , phényl, lauryl.

Le composé (A) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (B) peut être un composé polyglycérolé répondant à la formule (B') suivante :

CₘH₂ₘ₋₁ -O-[ Gly ]n-CₘH₂ₘ₋₁ (B')

dans laquelle m est un entier allant de 2 à 6, n est un entier allant de 2 à 200, de préférence allant de 2 à 100, de préférence allant de 2 à 50, de préférence n allant de 2 à 20, de préférence allant de 2 à 10, et préférentiellement allant de 2 à 5, et en particulier égal à 3 ; Gly désigne :

-CH₂-CH(OH)-CH₂-O- ou -CH₂-CH(CH₂OH)-O-

Avantageusement, la somme du nombre de groupements éthyléniques par molécule du composé (B) et du nombre d'atomes d'hydrogène liés à des atomes de silicium par molécule du composé (A) est d'au moins 4.

Il est avantageux que le composé (A) soit ajouté en une quantité telle que le rapport moléculaire entre la quantité totale d'atomes d'hydrogène liés à des atomes de silicium dans le composé (A) et la quantité totale de tous les groupements à insaturation éthylénique dans le composé (B) soit compris dans la gamme de 1/1 à 20/1.

Le composé (C) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C) est de préférence ajouté de 0,1 à 1000 parts en poids, mieux de 1 à 100 parts en poids, en tant que métal platine propre pour 1000 parts en poids de la quantité totale des composés (A) et (B).

L'élastomère de silicone polyglycérolé selon l'invention est généralement mélangé avec au moins une huile hydrocarbonée et/ou une huile siliconée pour former un gel.

Dans ces gels, l'élastomère polyglycérolé est souvent sous forme de particules non-sphériques.

De tels élastomères sont notamment décrits dans la demande de brevet W02004/024798.

Comme élastomères de silicone polyglycérolés, on peut utiliser ceux vendus sous les dénominations "KSG-710", "KSG-810", "KSG-820", "KSG-830", "KSG-840" par la société Shin Etsu.

L'émulsion selon l'invention comprend au moins un élastomère polyoxyalkyléné et un élastomère polyglycérolé.

Selon un aspect de l'invention, les élastomères de silicone émulsionnants peuvent être présents dans la composition selon l'invention en une teneur totale au moins supérieure à 3% en poids, par rapport au poids total de la composition, notamment allant de 3 à 15 % en poids, de préférence supérieure ou égale à 3,5 % en poids, notamment allant de 3,5 à 10 % en poids, et plus préférentiellement supérieure ou égale à 4 % en poids notamment allant de 4 à 7 % en poids.

Selon un autre aspect de l'invention, chaque élastomère émulsionnant peut être présent dans la composition selon l'invention en une teneur au moins supérieure à 1,5 % en poids, par rapport au poids total de la composition, et de préférence allant de 1,5 à 5 % en poids.

Selon un mode préféré de réalisation, l'émulsion selon l'invention peut comprendre au moins un élastomère polyoxyalkyléné en une teneur supérieure au moins supérieure à 1,5% en poids, par rapport au poids total de la composition, et de préférence, allant de 1,5 % à 5%, et un élastomère polyglycérolé en une teneur au moins supérieure à 1,5% en poids, par rapport au poids total de la composition, et de préférence, allant de 1,5 % à 5% en poids par rapport au poids total de la composition.

Selon encore un autre aspect, l'invention vise une composition cosmétique sous forme d'une émulsion eau-dans-huile comprenant :
i) au moins 1,5 % en poids par rapport au poids total de la composition d'un élastomère de silicone émulsionnant polyoxyalkylené ;
ii) au moins 1,5 % en poids par rapport au poids total de la composition d'un élastomère de silicone émulsionnant polyglycerolé ;
iii) au moins 20% en poids par rapport au poids total de la composition, d'eau ;
iv) au moins une huile volatile ;
v) au moins 5% en poids par rapport au poids total de la composition d'au moins un solvant organique miscible à l'eau choisi parmi les glycols et les polyols ;
vi) au moins 5% en poids par rapport au poids total de la composition d'au moins une matière colorante,
la composition ayant une viscosité allant de 10 Pa.s à 300 Pa.s pour un cisaillement de 1 s⁻¹.

### Elastomères additionnels non émulsionnants

Outre les élastomères émulsionnants, la composition selon l'invention peut comprendre des élastomères non émulsionnants.

Le terme élastomères de silicone "non émulsionnant" définit des élastomères organopolysiloxane ne contenant pas de chaîne hydrophile telle que des chaînes polyoxyalkylènes ou polyglycérolés.

L'élastomère de silicone non émulsionnant est un organopolysiloxane réticulé élastomère pouvant être obtenu par réaction d'addition réticulation de diorganopolysiloxane contenant au moins un hydrogène lié au silicium et de diorganopolysiloxane ayant des groupements à insaturation éthylénique liés au silicium, notamment en présence de catalyseur platine ; ou par réaction de condensation réticulation déhydrogénation entre un diorganopolysiloxane à terminaisons hydroxyle et un diorganopolysiloxane contenant au moins un hydrogène lié au silicium, notamment en présence d'un organoétain ; ou par réaction de condensation réticulation d'un diorganopolysiloxane à terminaisons hydroxyle et d'un organopolysilane hydrolysable ; ou par réticulation thermique d'organopolysiloxane, notamment en présence de catalyseur organopéroxyde ; ou par réticulation d'organopolysiloxane par radiations de haute énergie telles que rayons gamma, rayons ultraviolet, faisceau électronique.

De préférence, l'organopolysiloxane réticulé élastomère est obtenu par réaction d'addition réticulation (A2) de diorganopolysiloxane contenant au moins deux hydrogènes liés chacun à un silicium, et (B2) de diorganopolysiloxane ayant au moins deux groupements à insaturation éthylénique liés au silicium, notamment en présence (C2) de catalyseur platine, comme par exemple décrit dans la demande EP-A-295886.

En particulier, l'organopolysiloxane peut être obtenu par réaction de diméthylpolysiloxane à terminaisons diméthylvinylsiloxy et de méthylhydrogénopolysiloxane à terminaisons triméthylsiloxy, en présence de catalyseur platine.

Le composé (A2) est le réactif de base pour la formation d'organopolysiloxane élastomère et la réticulation s'effectue par réaction d'addition du composé (A2) avec le composé (B2) en présence du catalyseur (C2).

Le composé (A2) est avantageusement un diorganopolysiloxane ayant au moins deux groupes alkényles inférieur (par exemple en C2-C4) ; le groupe alkényle inférieur peut être choisi parmi les groupes vinyl, allyl, et propényl. Ces groupements alkényles inférieurs peuvent être situés en toute position de la molécule organopolysiloxane mais sont de préférence situés aux extrémités de la molécule organopolysiloxane. L'organopolysiloxane (A2) peut avoir une structure chaîne ramifiée, chaîne linéaire, cyclique ou réseau mais la structure chaîne linéaire est préférée. Le composé (A2) peut avoir une viscosité allant de l'état liquide à l'état de gomme. De préférence, le composé (A2) a une viscosité d'au moins 100 centistokes à 25 °C.

Les organopolysiloxanes (A2) peuvent être choisi parmi les méthylvinylsiloxanes, les copolymères méthylvinylsiloxane-diméthylsiloxane, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxane-méthylvinylsiloxane à terminaisons triméthylsiloxy, les méthyl(3,3,3-trifluoropropyl)polysiloxane à terminaisons diméthylvinylsiloxy, et les copolymères diméthylsiloxane-méthyl(3,3,3-trifluoropropyl)siloxane à terminaisons diméthylvinylsiloxy.

Le composé (B2) est en particulier un organopolysiloxane ayant au moins 2 hydrogènes liés au silicium dans chaque molécule et est donc le réticulant du composé (A2).
Avantageusement, la somme du nombre de groupements éthyléniques par molécule du composé (A2) et le nombre d'atomes d'hydrogène liés au silicum par molécule du composé (B2) est d'au moins 4.
Le composé (B2) peut être sous toute structure moléculaire, notamment de structure chaîne linéaire, chaîne ramifiée, structure cyclique.
Le composé (B2) peut avoir une viscosité à 25 °C allant de 1 à 50 000 centistokes, notamment pour être bien miscible avec le composé (A).
Il est avantageux que le composé (B2) soit ajouté en une quantité telle que le rapport moléculaire entre la quantité totale d'atomes d'hydrogène liés au silicium dans le composé (B2) et la quantité totale de tous les goupements à insaturation éthylénique dans le composé (A2) aille dans la gamme de 1/1 à 20/1.
Le composé (B2) peut être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane.

Le composé (C2) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C2) est de préférence ajouté de 0,1 à 1000 parts en poids, mieux de 1 à 100 parts en poids, en tant que métal platine propre pour 1000 parts en poids de la quantité totale des composés (A2) et (B2).

D'autres groupes organiques peuvent être liés au silicium dans les organopolysiloxane (A2) et (B2) décrits précédemment, comme par exemple des groupes alkyles tels que méthyl, éthyl, propyl, butyl, octyl ; des groupes alkyles substitués tels que 2-phényléthyl, 2-phénylpropyl, 3,3,3-trifluoropropyl ; des groupes aryles tels que phényl, tolyl, xylyl ; des groupes aryles substitutés tel que phényléthyl ; et des groupes hydrocarbonés monovalents substitués tels que un groupe époxy, un gropue ester carboxylate, un groupe mercapto.

L'élastomère de silicone non émulsionnant selon l'invention peut être mélangé avec au moins une huile hydrocarbonée et/ou une huile siliconée pour former un gel. Dans ces gels, l'élastomère non émulsionnant est sous forme de particules non-sphériques.

Comme élastomères non émulsionnants, on peut utiliser ceux vendus sous les dénominations "KSG-6", "KSG-15", "KSG-16", "KSG-18", "KSG-41", "KSG-42", "KSG-43", "KSG-44, USG-105, USG-106 par la société Shin Etsu, "DC 9040", "DC9041", "DC 9509", "DC9505", "DC 9506", "DC 5930", "DC9350", "DC9045", "DC9043" par la société Dow Corning, "GRANSIL" par la société Grant Industries, "SFE 839" par la société General Electric.

Les élastomères non émulsionnants, lorsqu'ils sont présents, peuvent être présents en une teneur allant de 0,01 % à 10 % en poids par rapport au poids total de la composition, de préférence allant de 0,5 % à 5 % en poids, et plus préférentiellement allant de 1 % à 3 % en poids.

### Les huiles

Selon un mode préféré de réalisation, les élastomère de silicone selon l'invention peuvent être mélangés avec au moins une huile (ou un mélange d'huiles) et forment ainsi un gel huileux.

L'huile peut être choisie parmi les huiles volatiles, les huiles non volatiles, et leurs mélanges.

La composition selon l'invention peut comprendre au moins une huile volatile.

Par « huile volatile », on entend au sens de l'invention toute huile susceptible de s'évaporer au contact de la peau, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40.000 Pa (0,001 à 300 mm de Hg) et de préférence allant de 1,3 à 1300 Pa (0,01 à 10 mm de Hg).

L'huile volatile peut être choisie parmi les huiles volatiles hydrocarbonées, les huiles volatiles siliconées, les huiles volatiles fluorées, et leurs mélanges.

On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permethyls®.

Comme huiles volatiles, on peut aussi utiliser les silicones volatiles, comme par exemple les huiles de silicones linéaires ou cycliques volatiles, notamment celles ayant une viscosité ≤ 5 centistokes (5 x 10⁻⁶ m²/s), et ayant notamment de 2 à 10 atomes de silicium, de préférence de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile fluorée n'a généralement pas de point éclair.
Comme huile volatile fluorée, on peut citer le nonafluoroéthoxybutane, le nonafluorométhoxybutane, le décafluoropentane, le tétradécafluorohexane, le dodécafluoropentane, et leurs mélanges.

Les huiles volatiles peuvent être présentes dans la composition en une teneur allant de 1 % à 50 % en poids par rapport au poids total de la composition, de préférence allant de 5 % à 30 % en poids et plus préférentiellement allant de 7 % à 20 % en poids par rapport au poids total de la composition.

La composition selon l'invention peut comprendre au moins une huile non volatile.

Par "huile non volatile", on entend une huile restant sur la peau à température ambiante et pression atmosphérique au moins plusieurs heures et ayant notamment une pression de vapeur inférieure à 0,13 Pa (0,01 mm de Hg).

Ces huiles non volatiles peuvent être des huiles hydrocarbonées notamment d'origine animale ou végétale, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810®, 812® et 818® par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le Parleam ®, le squalane, les huiles de paraffine, et leurs mélanges,
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le néopentanoate d'isodecyl, le palmitate de 2-éthyl-hexyle, l'isostéarate d'isostéaryle, le laurate de 2-hexyl-décyle, le palmitate de 2-octyl-décyle, le myristate de 2-octyl-dodécyle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle, le lactate de 2-octyl-dodécyle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et leurs mélanges.

Les huiles non volatiles peuvent être présentes dans la composition en une teneur allant de 1 % à 50 % en poids par rapport au poids total de la composition, de préférence allant de 5 % à 40 % en poids et plus préférentiellement allant de 10 % à 30 % en poids.

Selon un mode préféré de réalisation, la composition selon l'invention comprend au moins deux huiles de silicone de volatilités différentes.

Selon un mode plus préféré de réalisation, la composition selon l'invention comprend au moins une huile de silicone volatile et une huile de silicone non volatile.

Au total dans la composition, l'huile (ou le mélange d'huiles) peut être présente en une teneur allant de 5 % à 70 % en poids, par rapport au poids total de la composition, de préférence allant de 10 % à 60 % en poids, de préférence allant de 20 % à 50 % en poids.

### Phase aqueuse

La composition selon l'invention comprend une phase aqueuse.

La phase aqueuse comprend de l'eau. L'eau peut être une eau florale telle que l'eau de bleuet et/ou une eau minérale telle que l'eau de VITTEL, l'eau de LUCAS ou l'eau de LA ROCHE POSAY et/ou une eau thermale.

La phase aqueuse peut également comprendre des solvants organiques miscibles à l'eau (à température ambiante - 25 °C) comme par exemple les monoalcools ayant de 2 à 6 atomes de carbone tels que l'éthanol, l'isopropanol ;
les polyols ayant notamment de 2 à 20 atomes de carbones, de préférence ayant de 2 à 10 atomes de carbone, et préférentiellement ayant de 2 à 6 atomes de carbone, tels que le glycérol, le propylène glycol, le butylène glycol, le pentylène glycol, l'hexylène glycol, le dipropylène glycol, le diéthylène glycol ;
les éthers de glycol (ayant notamment de 3 à 16 atomes de carbone) tels que les alkyl(C₁-C₄)éther de mono, di- ou tripropylène glycol, les alkyl(C₁-C₄)éthers de mono, di- ou triéthylène glycol, et leurs mélanges.

Selon un mode préféré de réalisation, les solvants organiques miscibles à l'eau son présents dans la composition selon l'invention en une teneur allant de 3 à 15% en poids, par rapport au poids total de la composition, de préférence allant de 5 à 12% en poids.

La phase aqueuse peut comprendre en outre des agents de stabilisation, par exemple le chlorure de sodium, le dichlorure de magnésium et le sulfate de magnésium.

La phase aqueuse peut également comprendre tout composé hydrosoluble ou hydrodispersible compatible avec une phase aqueuse tels que des gélifiants, des polymères filmogènes, des épaississants, des tensioactifs et leurs mélanges.

De préférence, la phase aqueuse peut être présente dans la composition selon l'invention en une teneur supérieure ou égale à 25% en poids par rapport au poids total de la composition et inférieure à 50% en poids par rapport au poids total de la composition.

De préférence, l'eau peut être présente dans la composition selon l'invention en une teneur allant de 25 à 50% en poids par rapport au poids total de la composition, de préférence allant de 30 à 50% en poids.

Selon un mode particulier de réalisation, la phase aqueuse est présente en une teneur telle que le rapport pondéral de la phase huileuse sur la phase aqueuse est compris entre 0,5 et 2,5, et de préférence entre 0,7 et 2, et de préférence, entre 1 et 2.

### Charges

La composition selon l'invention peut comprendre des charges.

Par charges, il faut comprendre des particules de toute forme, incolores ou blanches, minérales ou de synthèse, insolubles dans le milieu de la composition quelle que soit la température à laquelle la composition est fabriquée.

Les charges peuvent être minérales ou organiques de toute forme, plaquettaires, sphériques ou oblongues, quelle que soit la forme cristallographique (par exemple feuillet, cubique, hexagonale, orthorombique, etc) . On peut citer le talc, le mica, la silice, le kaolin, les poudres de polyamide (Nylon®), les poudres de poly-β-alanine, les poudres de polyéthylène, les poudres de polyuréthane telle que la poudre de copolymère de diisocyanate d'hexaméthylène et de triméthylol hexyl lactone vendue sous les dénominations PLASTIC POWDER D-400 par la société TOSHIKI, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les poudres de résine de silicone, en particulier les poudres de silsesquioxane (poudres de résine de silicone notamment décrites dans le brevet EP 293795 ; Tospearls® de Toshiba, par exemple), les particules de polyorganosiloxanes élastomères, les particules de polyméthacrylate de méthyle, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium ; le sulfate de baryum et leurs mélanges.

Selon un mode préféré de réalisation, la composition selon l'invention comprend une charge sphérique. La charge sphérique est de préférence choisie parmi les poudres de polyamide ou les poudres de copolymère de diisocyanate d'hexaméthylène et de triméthylol hexyl lactone.

Les charges dispersées dans la composition peuvent être présentes en une teneur allant de 0,5 % à 20 % en poids, par rapport au poids total de la composition, de préférence allant de 1 % à 15 % en poids, et préférentiellement allant de 2 % à 10 % en poids.

### LES MATIERES COLORANTES

Selon un mode de réalisation particulier de l'invention, la composition peut comprendre au moins une matière colorante.

On entend par matière colorante au sens de la présente invention, un composé susceptible de produire un effet optique coloré lorsqu'il est formulé en quantité suffisante dans un milieu cosmétique approprié.

La matière colorante peut être notamment choisie parmi les pigments, les nacres, les paillettes, les colorants liposolubles, les colorants hydrosolubles, et leurs mélanges.
Par « pigments », il faut comprendre des particules blanches ou colorées, minérales ou organiques, insolubles dans la phase organique liquide, destinées à colorer et/ou opacifier la composition.
Par « nacres », il faut comprendre des particules irisées, notamment produites par certains mollusques dans leur coquille ou bien synthétisées, insolubles dans le milieu de la composition.
Par « colorants », il faut comprendre des composés généralement organiques solubles dans les corps gras comme les huiles ou dans une phase aqueuse.

Les matières colorantes peuvent être présentes en une teneur allant de 0,01% à 40 % en poids, par rapport au poids total de la composition, de préférence allant de 5 % à 30 % en poids, et préférentiellement allant de 5 à 20% en poids.

Selon un mode de réalisation de l'invention, la matière colorante comprend au moins un pigment.

Les pigments peuvent être choisis parmi les pigments minéraux, les pigments organiques, et les pigments composites (c'est-à-dire des pigments à base de matériaux minéraux et/ou organiques).

Par pigments, il faut comprendre des particules de toute forme, dotées d'un effet optique, minérales ou de synthèse, insolubles dans le milieu de la composition quelle que soit la température à laquelle la composition est fabriquée.

Les pigments peuvent être choisis parmi les pigments monochromes, les laques, les nacres, les pigments à effet optiques, comme les pigments réfléchissants et les pigments goniochromatiques.

Les pigments minéraux peuvent être choisis parmi les pigments d'oxyde métallique, le mica recouvert de dioxyde de titane, le mica recouvert d'oxychlorure de bismuth, le mica titane recouvert d'oxyde de fer, le mica-titane recouvert de bleu ferrique, le mica titane recouvert d'oxyde de chrome, les oxydes de fer, le dioxyde de titane, les oxydes de zinc, l'oxyde de cérium, l'oxyde de zirconium, l'oxyde de chrome ; le violet de manganèse, le bleu de prusse, le bleu outremer, le bleu ferrique, l'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth, et leurs mélanges.

Les pigments organiques peuvent être par exemple :
- le carmin de cochenille,
- les pigments organiques de colorants azoïques, anthraquinoniques, indigoïdes, xanthéniques, pyréniques, quinoliniques, de triphénylméthane, de fluorane ;
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane. Ces colorants comportent généralement au moins un groupe acide carboxylique ou sulfonique ;
- les pigments mélaniques.

Parmi les pigments organiques, on peut citer les D&C Blue n°4, D&C Brown n° 1, D&C Green n°5, D&C Green n°6, D&C Orange n°4, D&C Orange n°5 , D&C Orange n°10 , D&C Orange n°11 , D&C Red n°6, D&C Red n°7, D&C Red n°17, D&C Red n°21, D&C Red n°22, D&C Red n°27, D&C Red n°28, D&C Red n°30, D&C Red n°31, D&C Red n°33, D&C Red n°34, D&C Red n°36, D&C Violet n°2, D&C Yellow n°7, D&C Yellow n°8, D&C Yellow n°10, D&C Yellow n°11, FD&C Blue n° 1, FD&C Green n°3, FD&C Red n°40 , FD&C Yellow n°5, FD&C Yellow n°6.

Selon un mode préféré de réalisation, les pigments présents dans la composition selon l'invention sont des pigments enrobés hydrophobes.
Par pigments enrobés hydrophobes, on entend des pigments traités en surface avec un agent hydrophobe pour les rendre compatibles avec la phase grasse de l'émulsion, notamment pour qu'ils aient une bonne mouillabilité avec les huiles de la phase grasse. Ainsi, ces pigments traités sont bien dispersés dans la phase grasse.

Les pigments destinés à être enrobés peuvent être des pigments minéraux ou organiques décrits ci-dessus.

On utilise de préférence des pigments d'oxydes de fer ou de dioxyde de titane.

L'agent de traitement hydrophobe peut ëtre choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.
Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zin, de sodium, de potassium. L'acide aminé peut être par exemple la lysine, l'acide glutamique, l'alanine

Le terme alkyl mentionné dans les composés cités précédemment désigne notamment un groupe alkyle ayant de 1 à 30 atomes de carbone, de préférence ayant de 5 à 16 atomes de carbone.
Des pigments traités hydrophobes sont notamment décrits dans la demande EP-A-1086683.

Les pigments peuvent être présents dans la composition selon l'invention en une teneur allant de 2 à 40 % en poids, par rapport au poids total de la composition, et de préférence allant de 5 à 30 % en poids, et préférentiellement allant de 5 à 20% en poids.

Les colorants liposolubles sont par exemple le rouge Soudan, le D&C Red n° 17, le D&C Green n°6, le β-carotène, l'huile de soja, le brun Soudan, le D&C Yellow n° 11, le D&C Violet n° 2, le D&C orange n° 5, le jaune quinoléine, le rocou, les bromoacides.

Les colorants hydrosolubles sont par exemple le jus de betterave, le bleu de méthylène, le caramel.

### Additifs

La composition selon l'invention peut comprendre au moins un autre ingrédient cosmétique usuel pouvant être choisi notamment parmi les agents gélifiants et/ou épaississants hydrophiles ou lipophiles, les antioxydants, les parfums, les conservateurs, les neutralisants, les filtres solaires, les vitamines, les hydratants, les composés auto-bronzants, les actifs antirides, les émollients, les actifs hydrophiles ou lipophiles, les agents anti-pollution ou anti-radicaux libres, les sequestrants, les agents filmogènes, les tensioactifs non élastomères, les actifs dermorelaxants, les agents apaisants, les agents stimulant la synthèse de macromolécules dermiques ou épidermiques et/ou empêchant leur dégradation, les agents anti-glycation, les agents anti-irritants, les agents desquamants, les agents dépigmentants, anti-pigmentants, ou pro-pigmentants, les inhibiteurs de NO-synthase, les agents stimulant la prolifération des fibroblastes ou des kéranocytes et/ou la différentiation des kéranocytes, les agents agissant sur la microcirculation, les agents agissant sur le métabolisme énergétique des cellules, les agents cicatrisants, et leurs mélanges.

Selon un mode préféré de réalisation, la composition selon l'invention a une viscosité comprise entre 10 et 300 Pa.s pour un cisaillement de 1 s⁻¹, et de préférence, comprise entre 15 et 200 Pa.s pour un cisaillement de 1 s⁻¹.

La viscosité de la composition est mesurée à l'aide d'un rhéomètre à contrainte imposée, le Rhéostress RS 600 de la société Thermo. La mesure est effectuée à 25° C à l'aide d'un cône plan de 60 mm, angle 2° sablé avec un entrefer de 0,105 mm.

La présente invention est illustrée plus en détail dans les exemples suivants.

### Exemple 1 : Fond de Teint

On a préparé un fond de teint ayant la composition suivante :

| | |
|---|---|
| Elastomère de silicone émulsionnant polyglycérolé à 25% en poids dans polydimethylsiloxane (6 cst) *(1)* | 13 |
| Elastomère de silicone émulsionnant polyoxyalkyléné à 25% en poids dans polydimethylsiloxane (6 cst) *(2)* | 10 |
| Cyclopentadimethylsioxane *(3)* | 11 |
| Phenyl trimethicone *(4)* | 9 |
| Pigments | 8 |
| Poudre de Nylon 12 *(5)* | 3 |
| | |
| Propylène Glycol | 1 |
| Glycérine | 6 |
| Sulfate de Magnésium | 1 |
| Conservateur | qs |
| Eau | qsp |

| | |
|---|---|
| (1) *KSG-710 société Shin Etsu* (2) *KSG-210 société Shin Etsu* (3) *Dow Corning 245 Fluid société Dow Corning* (4) *Dow Corning 556 Cosmetic Grade Fluide société Dow Corning* (5) *Orgasol 2002 Exd Nat Cos 204 société Arkema* | |

### Mode de préparation :

On mélange l'élastomère de silicone émulsionnant polyglycérolé, l'élastomère de silicone émulsionnant polyoxyalkyléné, la polydimethylsiloxane, la cyclopentadimethylsioxane, la phenyl trimethicone, les pigments et la poudre de Nylon 12 (phase A).

On mélange séparément le propylène glycol, la glycérine, le sulfate de magnésium, les conservateurs et l'eau (phase B).

On verse la phase B dans la phase A pour former l'émulsion.

Le fond de teint obtenu présente une texture crémeuse, s'étale facilement sur la peau, procurant une sensation de douceur et de fondant.

### Exemple 2 : Fond de Teint

On a préparé un fond de teint ayant la composition suivante :

| | |
|---|---|
| Elastomère de silicone émulsionnant polyglycérolé à 25% en poids dans polydimethylsiloxane (6 cst) *(1)* | 13 |
| Elastomère de silicone émulsionnant polyoxyalkyléné à 25% en poids dans polydimethylsiloxane (6 cst) *(2)* | 9 |
| Cyclopentadimethylsioxane *(3)* | 13 |
| Phenyl trimethicone *(4)* | 6 |
| Pigments | 12 |
| Poudre de Nylon 12 *(5)* | 3 |
| | |
| Propylène Glycol | 2 |
| Glycérine | 5 |
| Sulfate de Magnésium | 1 |
| Conservateur | qs |
| Eau | qsp |

| | |
|---|---|
| *(1) KSG-710 société Shin Etsu* *(2) KSG-210 société Shin Etsu* *(3) Dow Corning 245 Fluid société Dow Corning* *(4) Dow Corning 556 Cosmetic Grade Fluide société Dow Corning* *(5) Orgasol 2002 Exd Nat Cos 204 société Arkema* | |

Le mode de préparation est le même que celui de l'exemple 1.

Le fond de teint obtenu présente une texture crémeuse, s'étale facilement sur la peau, procurant une sensation de douceur et de fondant.

### Exemple 3 : Fond de Teint

On a préparé un fond de teint ayant la composition suivante :

| | |
|---|---|
| Elastomère de silicone émulsionnant polyglycérolé à 25% en poids dans polydimethylsiloxane (6 cst) *(1)* | 8,5 |
| Elastomère de silicone émulsionnant polyoxyalkyléné à 25% en poids dans polydimethylsiloxane (6 cst) *(2)* | 10 |
| Polydimethylsioxane oxyéthyléné *(3)* | 2 |
| Cyclopentadimethylsioxane *(4)* | 17 |
| polydimethylsiloxane (5 cst) *(5)* | 5 |
| Pigments | 12 |
| Poudre de copolymère de diisocyanate d'hexaméthylène et de triméthylol hexyl lactone *(6)* | 3 |
| | |
| Propylène Glycol | 2 |
| Glycérine | 5 |
| Sulfate de Magnésium | 1 |
| Conservateur | qs |
| Eau | qsp |

| | |
|---|---|
| *(1) KSG-710 par la société Shin Etsu* *(2) KSG-210 par la société Shin Etsu* *(3) KF-6017 par la société Shin Etsu* *(4) Dow Corning 245 Fluid par la société Dow Corning* *(5) Dow Corning Fluid 200 5 cs par la société Dow Corning* *(6) PLASTIC POWDER D-400 par la société TOSHIKI* | |

### Mode de préparation :

On mélange l'élastomère de silicone émulsionnant polyglycérolé, l'élastomère de silicone émulsionnant polyoxyalkyléné, la polydimethylsiloxane, la cyclopentadimethylsioxane, la Polydimethylsioxane oxyéthyléné, les pigments et la poudre de Nylon 12 (phase A).

On mélange séparément le propylène glycol, la glycérine, le sulfate de magnésium, les conservateurs et l'eau (phase B).

On verse la phase B dans la phase A pour former l'émulsion.

Le fond de teint obtenu présente une texture crémeuse, s'étale facilement sur la peau, procurant une sensation de douceur et de fondant.

## Revendications

1. Composition cosmétique sous forme d'émulsion eau-dans-huile comprenant au moins deux élastomères de silicone émulsionnants différents, l'un étant un élastomère de silicone polyglycérolé et l'autre étant un élastomère de silicone polyoxyalkyléné, la teneur totale en élastomères de silicone émulsionnants présents dans la composition étant au moins supérieure ou égale à 3 % en poids, par rapport au poids total de la composition.

2. Composition cosmétique selon la revendication 1, **caractérisée en ce que** les élastomères de silicone émulsionnants sont présents en une teneur totale allant de 3% à 15% en poids, par rapport au poids total de la composition, de préférence allant de 3,5% à 10% en poids, et plus préférentiellement allant de 4 à 7% en poids.

3. Composition cosmétique selon la revendication 1 ou 2, **caractérisée en ce que** chaque élastomère de silicone émulsionnant est présent dans la composition en une teneur supérieure à 1,5% en poids, par rapport au poids total de la composition.

4. Composition selon la revendication 1, **caractérisée en ce que** les élastomères de silicones polyoxyalkylénés sont obtenus par réaction d'addition réticulation de diorganosiloxane contenant au moins un hydrogène lié au silicium et un polyoxyalkylène ayant au moins deux groupements à insaturation éthylénique.

5. Composition selon la revendication 1, **caractérisée en ce que** les élastomères de silicones polyglycérolés sont obtenus par réaction d'addition réticulation de diorganosiloxane contenant au moins un hydrogène lié au silicium et de composés polyglycérolés ayant des groupements à insaturation.éthylénique, notamment en présence de catalyseur platine.

6. Composition, selon la revendication 1, **caractérisée en ce que** le ou les élastomères de silicone polyglycérolés sont présents en une teneur au moins supérieure à 1,5 % en poids, par rapport au poids total de la composition, notamment allant de 1,5 à 5 % en poids.

7. Composition, selon la revendication 1, **caractérisée en ce que** les élastomères de silicone polyoxyalkylénés sont présents en une teneur au moins supérieure à 1,5 % en poids, par rapport au poids total de la composition, notamment allant de 1,5 à 5 % en poids.

8. Composition cosmétique selon l'une quelconque des revendications précédentes, **caractérisé en ce qu'**elle comprend au moins une huile choisie parmi les huiles hydrocarbonées et les huile siliconées.

9. Composition selon l'une quelconque des revendications précédentes, **caractérisée en ce qu'**elle contient au moins une huile volatile.

10. Composition selon la revendication 9 **caractérisée en ce que** l'huile volatile est choisie parmi les huiles volatiles hydrocarbonées, les huiles volatiles siliconées, les huiles volatiles fluorées, et leurs mélanges.

11. Composition selon les revendications 9 et 10, **caractérisée en ce que** les huiles volatiles sont présentes en une teneur allant de 1% à 50 % en poids par rapport au poids total de la composition, de préférence allant de 5 % à 30 % en poids, et plus préférentiellement de 7 % à 20 % en poids.

12. Composition selon l'une quelconque des revendications précédentes, **caractérisée en ce qu'**elle contient une huile non volatile.

13. Composition selon la revendication précédente, **caractérisée en ce que** l'huile non volatile est présente en une teneur allant de 1% à 50 % en poids par rapport au poids total de la composition, de préférence allant de 5 % à 40 % en poids, et plus préférentiellement de 10 % à 30 % en poids.

14. Composition selon l'une quelconque des revendications précédentes, **caractérisée en ce que** l'huile (ou les huiles) est (sont) présente(s) en une teneur totale allant de 5% à 70 % en poids par rapport au poids total de la composition, de préférence allant de 10 % à 60 % en poids, et plus préférentiellement de 20 % à 50 % en poids.

15. Composition selon l'une quelconque des revendications précédentes, **caractérisée en ce qu'**elle comprend une phase aqueuse en une teneur allant de 25% à 50% en poids, par rapport au poids total de la composition, et notamment de 30 % à 50 % en poids.

16. Composition selon l'une quelconque des revendications précédente **caractérisée en ce qu'**elle contient au moins un solvant organique miscible à l'eau.

17. Composition selon la revendication précédente, **caractérisée en ce que** le solvant organique miscible à l'eau est choisi parmi les polyols et les esters de glycols.

18. Composition selon les revendications 16 et 17, **caractérisée en ce** les solvants organiques miscibles à l'eau son présents dans la composition selon l'invention en une teneur d'au moins 3 % en poids par rapport au poids total de la composition, de préférence allant de 3 à 15% en poids, et de préférence encore, allant de 5 % à 12% en poids.

19. Composition selon l'une quelconque des revendications précédentes, **caractérisée en ce que** le rapport pondéral de la phase huileuse sur la phase aqueuse va de 0,5 et 2,5, de préférence de 0,7 à 2, et plus préférentiellement de 1 à 2.

20. Composition selon l'une quelconque des revendications précédentes, **caractérisée en ce qu'**elle comprend au moins une charge.

21. Composition selon la revendication précédente, **caractérisée en ce** la charge est choisie parmi les charges minérales et organiques, sous forme plaquettaires, sphériques ou oblongues.

22. Composition selon la revendication précédente, **caractérisée en ce qu'**elle comprend au moins une charge sphérique.

23. Composition selon la revendication précédente, **caractérisée en ce** la charge est présente en une teneur allant de 0,5 à 20% en poids par rapport au poids total de la composition, de préférence allant de 1 à 15% en poids, et plus préférentiellement allant de 2 à 10% en poids.

24. Composition selon l'une quelconque des revendications précédentes, **caractérisée en ce qu'**elle comprend au moins une matière colorante choisie parmi les pigments, les nacres, les paillettes, les colorants liposolubles, les colorants hydrosolubles, et leurs mélanges.

25. Composition selon l'une quelconque des revendications précédentes, **caractérisée en ce qu'**elle comprend des pigments.

26. Composition selon la revendication précédente, **caractérisée en ce que** le pigment est un pigment enrobé.

27. Composition selon la revendication précédente, **caractérisé en ce que** le pigment enrobé est choisi parmi les pigments d'oxydes de fer ou de dioxyde de titane.

28. Composition selon l'une quelconque des revendications 24 à 27, **caractérisée en ce** la matière colorante est présente en une teneur allant de 0,01 à 40% en poids par rapport au poids total de la composition, de préférence allant de 5 à 30 % en poids, et plus préférentiellement allant de 5 à 20% en poids.

29. Composition selon l'une quelconque des revendications précédentes, **caractérisée en ce qu'**elle contient au moins un élastomère non émulsionnant.

30. Composition selon la revendication précédente, **caractérisée en ce que** l'élastomère non émulsionnant est présent en une teneur allant de 0,01 % à 10 % en poids par rapport au poids total de la composition, de préférence allant de 0,5 % à 5 % en poids, et plus préférentiellement de 1 % à 3 % en poids.

31. Composition selon l'une quelconque des revendications précédentes, **caractérisée en ce qu'**elle présente une viscosité comprise allant de 10 Pa.s à 300 Pa.s, et de préférence, allant de 15 à 200 Pa.s pour un cisaillement de 1s⁻¹.

32. Composition selon l'une quelconque des revendications précédentes, **caractérisée en ce qu'**elle comprend au moins un ingrédient cosmétique choisi parmi les tensioactifs non élastomères, les agents gélifiants et/ou épaississants hydrophiles ou lipophiles, les antioxydants, les parfums, les conservateurs, les neutralisants, les filtres solaires, les vitamines, les hydratants, les composés auto-bronzants, les actifs antirides, les émollients, les actifs hydrophiles ou lipophiles, les agents anti-pollution ou anti-radicaux libres, les sequestrants, les agents filmogènes, les actifs dermorelaxants, les agents apaisants, les agents stimulant la synthèse de macromolécules dermiques ou épidermiques et/ou empêchant leur dégradation, les agents anti-glycation, les agents anti-irritants, les agents desquamants, les agents dépigmentants, anti-pigmentants, ou pro-pigmentants, les inhibiteurs de NO-synthase, les agents stimulant la prolifération des fibroblastes ou des kéranocytes et/ou la différentiation des kéranocytes, les agents agissant sur la microcirculation, les agents agissant sur le métabolisme énergétique des cellules, les agents cicatrisants, et leurs mélanges.

33. Composition cosmétique sous forme d'une émulsion eau-dans-huile comprenant :
i) au moins 1,5 % en poids par rapport au poids total de la composition d'un élastomère de silicone émulsionnant polyoxyalkylené ;
ii) au moins 1,5 % en poids par rapport au poids total de la composition d'un élastomère de silicone émulsionnant polyglycerolé ;
iii) au moins 20% en poids par rapport au poids total de la composition, d'eau ;
iv) au moins une huile volatile ;
v) au moins 5% en poids par rapport au poids total de la composition d'au moins un solvant organique miscible à l'eau choisi parmi les glycols et les polyols ;
vi) au moins 5% en poids par rapport au poids total de la composition d'au moins une matière colorante, la composition ayant une viscosité allant de 10 Pa.s à 300 Pa.s pour un cisaillement de 1s⁻¹.

34. Procédé cosmétique de maquillage ou de soin des matières kératiniques comprenant l'application sur lesdites matières kératiniques d'une composition selon l'une quelconque des revendications précédentes.

35. Utilisation d'une composition selon l'une quelconque des revendications 1 à 33 pour obtenir un maquillage confortable et/ou frais et/ou sans sensation de collant et/ou s'étalant facilement.

## Claims

1. Cosmetic composition in the form of a water-in-oil emulsion comprising at least two different emulsifying silicone elastomers, one being a polyglycerolated silicone elastomer and the other being a polyoxyalkylenated silicone elastomer, the total content of emulsifying silicone elastomers present in the composition being at least greater than or equal to 3% by weight, relative to the total weight of the composition.

2. Cosmetic composition according to Claim 1, **characterized in that** the emulsifying silicone elastomers are present in a total content ranging from 3% to 15% by weight, relative to the total weight of the composition, preferably ranging from 3.5% to 10% by weight, and more preferentially ranging from 4% to 7% by weight.

3. Cosmetic composition according to Claim 1 or 2, **characterized in that** each emulsifying silicone elastomer is present in the composition in a content of greater than 1.5% by weight relative to the total weight of the composition.

4. Composition according to Claim 1, **characterized in that** the polyoxyalkylenated silicone elastomers are obtained by crosslinking addition reaction of diorganosiloxane containing at least one hydrogen linked to silicon and a polyoxyalkylene having at least two ethylenically unsaturated groups.

5. Composition according to Claim 1, **characterized in that** the polyglycerolated silicone elastomers are obtained by crosslinking addition reaction of diorganosiloxane containing at least one hydrogen linked to silicon and of polyglycerolated compounds having ethylenically unsaturated groups, in particular in the presence of a platinum catalyst.

6. Composition according to Claim 1, **characterized in that** the polyglycerolated silicone elastomer(s) is (are) present in a content of at least greater than 1.5% by weight, relative to the total weight of the composition, in particular ranging from 1.5% to 5% by weight.

7. Composition according to Claim 1, **characterized in that** the polyoxyalkylenated silicone elastomers are present in a content of at least greater than 1.5% by weight, relative to the total weight of the composition, in particular ranging from 1.5% to 5% by weight.

8. Cosmetic composition according to any one of the preceding claims, **characterized in that** it comprises at least one oil chosen from hydrocarbon-based oils and silicone oils.

9. Composition according to any one of the preceding claims, **characterized in that** it contains at least one volatile oil.

10. Composition according to Claim 9, **characterized in that** the volatile oil is chosen from volatile hydrocarbon-based oils, volatile silicone oils and volatile fluoro oils, and mixtures thereof.

11. Composition according to Claims 9 and 10,
**characterized in that** the volatile oils are present in a content ranging from 1% to 50% by weight, relative to the total weight of the composition, preferably ranging from 5% to 30% by weight, and more preferentially from 7% to 20% by weight.

12. Composition according to any one of the preceding claims, **characterized in that** it contains a non-volatile oil.

13. Composition according to the preceding claim, **characterized in that** the non-volatile oil is present in a content ranging from 1% to 50% by weight, relative to the total weight of the composition, preferably ranging from 5% to 40% by weight, and more preferentially from 10% to 30% by weight.

14. Composition according to any one of the preceding claims, **characterized in that** the oil (or oils) is (are) present in a total content ranging from 5% to 70% by weight, relative to the total weight of the composition, preferably ranging from 10% to 60% by weight, and more preferentially from 20% to 50% by weight.

15. Composition according to any one of the preceding claims, **characterized in that** it comprises an aqueous phase in a content ranging from 25% to 50% by weight, relative to the total weight of the composition, and in particular from 30% to 50% by weight.

16. Composition according to any one of the preceding claims, **characterized in that** it contains at least one water-miscible organic solvent.

17. Composition according to the preceding claim, **characterized in that** the water-miscible organic solvent is chosen from polyols and glycol esters.

18. Composition according to Claims 16 and 17, **characterized in that** the water-miscible organic solvents are present in the composition according to the invention in a content of at least 3% by weight, relative to the total weight of the composition, preferably ranging from 3% to 15% by weight and more preferably ranging from 5% to 12% by weight.

19. Composition according to any one of the preceding claims, **characterized in that** the weight ratio of the oily phase to the aqueous phase ranges from 0.5 to 2.5, preferably from 0.7 to 2 and more preferentially from 1 to 2.

20. Composition according to any one of the preceding claims, **characterized in that** it comprises at least one filler.

21. Composition according to the preceding claim, **characterized in that** the filler is chosen from inorganic and organic fillers, which are plate-shaped, spherical or oblong.

22. Composition according to the preceding claim, **characterized in that** it comprises at least one spherical filler.

23. Composition according to the preceding claim, **characterized in that** the filler is present in a content ranging from 0.5% to 20% by weight, relative to the total weight of the composition, preferably ranging from 1% to 15% by weight, and more preferentially ranging from 2% to 10% by weight.

24. Composition according to any one of the preceding claims, **characterized in that** it comprises at least one dyestuff chosen from pigments, pearlescent agents, flakes, liposoluble dyes and water-soluble dyes, and mixtures thereof.

25. Composition according to any one of the preceding claims, **characterized in that** it comprises pigments.

26. Composition according to the preceding claim, **characterized in that** the pigment is a coated pigment.

27. Composition according to the preceding claim, **characterized in that** the coated pigment is chosen from iron oxide or titanium dioxide pigments.

28. Composition according to any one of Claims 24 to 27, **characterized in that** the dyestuff is present in a content ranging from 0.01% to 40% by weight, relative to the total weight of the composition, preferably ranging from 5% to 30% by weight and more preferentially ranging from 5% to 20% by weight.

29. Composition according to any one of the preceding claims, **characterized in that** it contains at least one non-emulsifying elastomer.

30. Composition according to the preceding claim, **characterized in that** the non-emulsifying elastomer is present in a content ranging from 0.01% to 10% by weight, relative to the total weight of the composition, preferably ranging from 0.5% to 5% by weight and more preferentially from 1% to 3% by weight.

31. Composition according to any one of the preceding claims, **characterized in that** it has a viscosity ranging from 10 Pa.s to 300 Pa.s, and preferably ranging from 15 to 200 Pa.s, for a shear of 1 s⁻¹.

32. Composition according to any one of the preceding claims, **characterized in that** it comprises at least one cosmetic ingredient chosen from non-elastomeric surfactants, hydrophilic or lipophilic gelling agents and/or thickeners, antioxidants, fragrances, preservatives, neutralizers, sunscreens, vitamins, moisturizers, self-tanning compounds, antiwrinkle active agents, emollients, hydrophilic or lipophilic active agents, anti-pollution agents or free-radical scavengers, sequestering agents, film-forming agents, dermo-relaxing active agents, calmatives, agents for stimulating the synthesis of dermal or epidermal macromolecules and/or for preventing degradation thereof, anti-glycation agents, anti-irritants, desquamating agents, depigmenting, antipigmenting or propigmenting agents, NO-synthase inhibitors, agents for stimulating fibroblast or keratinocyte proliferation and/or keratinocyte differentiation, agents for acting on the microcirculation, agents for acting on the energy metabolism of cells, and cicatrizing agents, and mixtures thereof.

33. Cosmetic composition in the form of a water-in-oil emulsion comprising:
i) at least 1.5% by weight, relative to the total weight of the composition, of a polyoxyalkylenated emulsifying silicone elastomer;
ii) at least 1.5% by weight, relative to the total weight of the composition, of a polyglycerolated emulsifying silicone elastomer;
iii) at least 20% by weight, relative to the total weight of the composition, of water;
iv) at least one volatile oil;
v) at least 5% by weight, relative to the total weight of the composition, of at least one water-miscible organic solvent chosen from glycols and polyols;
vi) at least 5% by weight, relative to the total weight of the composition, of at least one dyestuff, the composition having a viscosity ranging from 10 Pa.s to 300 Pa.s, for a shear of 1 s⁻¹.

34. Cosmetic process for making up or caring for keratin materials, comprising the application to said keratin materials of a composition according to any one of the preceding claims.

35. Use of a composition according to any one of Claims 1 to 33, for obtaining a makeup which is comfortable and/or fresh and/or does not have a sticky feeling and/or spreads easily.

## Patentansprüche

1. Kosmetische Zusammensetzung in Form einer Wasser-in-Öl-Emulsion, die mindestens zwei unterschiedliche emulgierende Siliconelastomere enthält, wobei eines ein mehrfach mit Glycerin verethertes Siliconelastomer und das andere ein polyalkoxyliertes Siliconelastomer ist und wobei der Gesamtgehalt der in der Zusammensetzung enthaltenen emulgierenden Siliconelastomere zumindest größer oder gleich 3 Gew.-%, bezogen auf das Gesamtgewicht der Zusammensetzung, ist.

2. Kosmetische Zusammensetzung nach Anspruch 1, **dadurch gekennzeichnet, dass** die emulgierenden Siliconelastomere in einer Gesamtmenge von 3 bis 15 Gew.-%, bezogen auf das Gesamtgewicht der Zusammensetzung, vorzugsweise 3,5 bis 10 Gew.-% und noch bevorzugter 4 bis 7 Gew.-% enthalten sind.

3. Kosmetische Zusammensetzung nach Anspruch 1 oder 2, **dadurch gekennzeichnet, dass** jedes emulgierende Siliconelastomer in der Zusammensetzung in einer Menge über 1,5 Gew.-%, bezogen auf das Gesamtgewicht der Zusammensetzung, enthalten ist.

4. Zusammensetzung nach Anspruch 1, **dadurch gekennzeichnet, dass** die polyalkoxylierten Siliconelastomere durch Vernetzung eines Diorganosiloxans, das mindestens ein an ein Siliciumatom gebundenes Wasserstoffatom enthält, und einem Polyoxyalkylen hergestellt sind, das mindestens zwei ethylenisch ungesättigte Gruppen aufweist.

5. Zusammensetzung nach Anspruch 1, **dadurch gekennzeichnet, dass** die mehrfach mit Glycerin veretherten Siliconelastomere durch Vernetzung eines Diorganosiloxans, das mindestens ein an ein Siliciumatom gebundenes Wasserstoffatom enthält, und mehrfach mit Glycerin veretherten Verbindungen, die ethylenisch ungesättigte Gruppen aufweisen, insbesondere in Gegenwart eines Platinkatalysators hergestellt sind.

6. Zusammensetzung nach Anspruch 1, **dadurch gekennzeichnet, dass** das oder die mehrfach mit Glycerin veretherten Siliconelastomere in einem Mengenanteil von zumindest über 1,5 Gew.-%, bezogen auf das Gesamtgewicht der Zusammensetzung, besonders im Bereich von 1,5 bis 5 Gew.-% enthalten sind.

7. Zusammensetzung nach Anspruch 1, **dadurch gekennzeichnet, dass** die polyalkoxylierten Siliconelastomere in einem Mengenanteil von zumindest über 1,5 Gew.-%, bezogen auf das Gesamtgewicht der Zusammensetzung, und insbesondere im Bereich von 1,5 bis 5 Gew.-% enthalten sind.

8. Kosmetische Zusammensetzung nach einem der vorhergehenden Ansprüche, **dadurch gekennzeichnet, dass** sie mindestens ein Öl enthält, das unter den Kohlenwasserstoffölen und Siliconölen ausgewählt ist.

9. Zusammensetzung nach einem der vorhergehenden Ansprüche, **dadurch gekennzeichnet, dass** sie mindestens ein flüchtiges Öl enthält.

10. Zusammensetzung nach Anspruch 9, **dadurch gekennzeichnet, dass** das flüchtige Öl unter den flüchtigen Kohlenwasserstoffölen, flüchtigen Siliconölen, flüchtigen fluorierten Ölen und deren Gemischen ausgewählt ist.

11. Zusammensetzung nach den Ansprüchen 9 und 10, **dadurch gekennzeichnet, dass** die flüchtigen Öle in einem Mengenanteil von 1 bis 50 Gew.-%, bezogen auf das Gesamtgewicht der Zusammensetzung, vorzugsweise 5 bis 30 Gew.-% und noch bevorzugter 7 bis 20 Gew.-% enthalten sind.

12. Zusammensetzung nach einem der vorhergehenden Ansprüche, **dadurch gekennzeichnet, dass** sie ein nichtflüchtiges Öl enthält.

13. Zusammensetzung nach dem vorhergehenden Anspruch, **dadurch gekennzeichnet, dass** das nichtflüchtige Öl in einem Mengenanteil von 1 bis 50 Gew.-%, bezogen auf das Gesamtgewicht der Zusammensetzung, vorzugsweise im Bereich von 5 bis 40 Gew.-% und noch bevorzugter 10 bis 30 Gew.-% enthalten ist.

14. Zusammensetzung nach einem der vorhergehenden Ansprüche, **dadurch gekennzeichnet, dass** das Öl (oder die Öle) in einer Gesamtmenge von 5 bis 70 Gew.-%, bezogen auf das Gesamtgewicht der Zusammensetzung, vorzugsweise 10 bis 60 Gew.-% und noch bevorzugter 20 bis 50 Gew.-% enthalten ist (sind).

15. Zusammensetzung nach einem der vorhergehenden Ansprüche, **dadurch gekennzeichnet, dass** sie eine wässrige Phase in einem Anteil von 25 bis 50 Gew.-%, bezogen auf das Gesamtgewicht der Zusammensetzung, und insbesondere 30 bis 50 Gew.-% enthält.

16. Zusammensetzung nach einem der vorhergehenden Ansprüche, **dadurch gekennzeichnet, dass** sie mindestens ein mit Wasser mischbares, organisches Lösungsmittel enthält.

17. Zusammensetzung nach dem vorhergehenden Anspruch, **dadurch gekennzeichnet, dass** das organische, mit Wasser mischbare Lösungsmittel unter den Polyolen und Glycolestern ausgewählt ist.

18. Zusammensetzung nach den Ansprüchen 16 und 17, **dadurch gekennzeichnet, dass** die mit Wasser mischbaren, organischen Lösungsmittel in der erfindungsgemäßen Zusammensetzung in einem Mengenanteil von mindestens 3 Gew.-%, bezogen auf das Gesamtgewicht der Zusammensetzung, vorzugsweise 3 bis 15 Gew.-% und noch bevorzugter 5 bis 12 Gew.-% enthalten sind.

19. Zusammensetzung nach einem der vorhergehenden Ansprüche, **dadurch gekennzeichnet, dass** das Gewichtsverhältnis der Ölphase und der wässrigen Phase im Bereich von 0,5 bis 2,5, vorzugsweise 0,7 bis 2 und noch bevorzugter 1 bis 2 liegt.

20. Zusammensetzung nach einem der vorhergehenden Ansprüche, **dadurch gekennzeichnet, dass** sie mindestens einen Füllstoff enthält.

21. Zusammensetzung nach dem vorhergehenden Anspruch, **dadurch gekennzeichnet, dass** der Füllstoff unter den anorganischen und organischen Füllstoffen in Form von plättchenförmigen Füllstoffen, sphärischen Füllstoffen oder länglichen Füllstoffen ausgewählt ist.

22. Zusammensetzung nach dem vorhergehenden Anspruch, **dadurch gekennzeichnet, dass** sie mindestens einen sphärischen Füllstoff enthält.

23. Zusammensetzung nach dem vorhergehenden Anspruch, **dadurch gekennzeichnet, dass** der Füllstoff in einem Mengenanteil von 0,5 bis 20 Gew.-%, bezogen auf das Gesamtgewicht der Zusammensetzung, vorzugsweise 1 bis 15 Gew.-% und noch bevorzugter 2 bis 10 Gew.-% enthalten ist.

24. Zusammensetzung nach einem der vorhergehenden Ansprüche, **dadurch gekennzeichnet, dass** sie mindestens ein Farbmittel enthält, das unter den Pigmenten, Perlglanzpigmenten, Pailletten, fettlöslichen Farbstoffen, wasserlöslichen Farbstoffen und deren Gemischen ausgewählt ist.

25. Zusammensetzung nach einem der vorhergehenden Ansprüche, **dadurch gekennzeichnet, dass** sie Pigmente enthält.

26. Zusammensetzung nach dem vorhergehenden Anspruch, **dadurch gekennzeichnet, dass** es sich bei dem Pigment um ein umhülltes Pigment handelt.

27. Zusammensetzung nach dem vorhergehenden Anspruch, **dadurch gekennzeichnet, dass** das umhüllte Pigment unter den Pigmenten von Eisenoxiden oder Titandioxid ausgewählt ist.

28. Zusammensetzung nach einem der Ansprüche 24 bis 27, **dadurch gekennzeichnet, dass** das Farbmittel in einem Mengenanteil von 0,01 bis 40 Gew.-%, bezogen auf das Gesamtgewicht der Zusammensetzung, vorzugsweise im Bereich von 5 bis 30 Gew.-% und insbesondere im Bereich von 5 bis 20 Gew.-% enthalten ist.

29. Zusammensetzung nach einem der vorhergehenden Ansprüche, **dadurch gekennzeichnet, dass** sie mindestens ein nichtemulgierendes Elastomer enthält.

30. Zusammensetzung nach dem vorhergehenden Anspruch, **dadurch gekennzeichnet, dass** das nichtemulgierende Elastomer in einem Mengenanteil von 0,01 bis 10 Gew.-%, bezogen auf das Gesamtgewicht der Zusammensetzung, vorzugsweise 0,5 bis 5 Gew.-% und noch bevorzugter 1 bis 3 Gew.-% vorliegt.

31. Zusammensetzung nach einem der vorhergehenden Ansprüche, **dadurch gekennzeichnet, dass** sie eine Viskosität im Bereich von 10 bis 300 Pa.s und vorzugsweise im Bereich von 15 bis 200 Pa.s für eine Scherung von 1 s⁻¹ aufweist.

32. Zusammensetzung nach einem der vorhergehenden Ansprüche, **dadurch gekennzeichnet, dass** sie mindestens einen kosmetischen Bestandteil enthält, der unter den nicht elastomeren grenzflächenaktiven Stoffen, hydrophilen oder lipophilen Gelbildnern und/oder Verdickungsmitteln, Antioxidantien, Parfums, Konservierungsmitteln, Neutralisationsmitteln, Sonnenschutzfiltern, Vitaminen, Hydratisierungsmitteln, selbstbräunenden Verbindungen, Wirkstoffen gegen Falten, Emollientien, hydrophilen oder lipophilen Wirkstoffen, Antipollutionsstoffen oder Radikalfängern für freie Radikale, Maskierungsmitteln, Filmbildnern, Dermo-Relaxantien, beruhigenden Stoffen, Stoffen, die die Synthese von Makromolekülen der Dermis oder Epidermis stimulieren und/oder ihre Zersetzung verhindern, Antiglycationswirkstoffen, reizlindernden Wirkstoffen, abschuppenden Wirkstoffen, Depigmentierungsmitteln, pigmentierungsverhindernden Wirkstoffen oder pigmentierungsfördernden Wirkstoffen, Inhibitoren der NO-Synthase, Wirkstoffen, die die Proliferation von Fibroblasten oder Keratinocyten und/oder die Differenzierung von Keratinocyten stimulieren, Wirkstoffen, die auf die Mikrozirkulation einwirken, Wirkstoffen, die auf den Energiehaushalt der Zellen einwirken, Wundheilungsmitteln und deren Gemischen ausgewählt ist.

33. Kosmetische Zusammensetzung in Form einer Wasser-in-Öl-Emulsion, die enthält:
i) mindestens 1,5 Gew.-% eines polyalkoxylierten emulgierenden Siliconelastomers, bezogen auf das Gesamtgewicht der Zusammensetzung;
ii) mindestens 1,5 Gew.-% eines mehrfach mit Glycerin veretherten emulgierenden Siliconelastomers, bezogen auf das Gesamtgewicht der Zusammensetzung;
iii) mindestens 20 Gew.-% Wasser, bezogen auf das Gesamtgewicht der Zusammensetzung;
iv) mindestens ein flüchtiges Öl;
v) mindestens 5 Gew.-% mindestens eines mit Wasser mischbaren, organischen Lösungsmittels, das unter den Glycolen und Polyolen ausgewählt ist, bezogen auf das Gesamtgewicht der Zusammensetzung;
vi) mindestens 5 Gew.-% mindestens eines Farbmittels, bezogen auf das Gesamtgewicht der Zusammensetzung,
wobei die Zusammensetzung für eine Scherung von 1 s⁻¹ eine Viskosität im Bereich von 10 bis 300 Pa.s besitzt.

34. Kosmetisches Verfahren zum Schminken oder für die Pflege von Keratinsubstanzen, das das Aufbringen einer Zusammensetzung nach einem der vorhergehenden Ansprüche auf die Keratinsubstanzen umfasst.

35. Verwendung einer Zusammensetzung nach einem der Ansprüche 1 bis 33, um eine angenehm zu tragende Schminke und/oder eine frische Schminke und/oder eine Schminke ohne klebriges Gefühl und/oder eine Schminke, die sich leicht verteilen lässt, zu bilden.
